# EUROPÄISCHE PATENTSCHRIFT

(11) **EP 0 695 558 B1**
(45) Veröffentlichungstag und Bekanntmachung des Hinweises auf die Patenterteilung: **16.07.2003**
(21) Anmeldenummer: 95250119.5
(22) Anmeldetag: 18.05.1995
(51) Int. Cl.: A61N 1/372

(54) **Telemetrievorrichtung, insbesondere für ein Gewebestimulator-System**
Telemetry device, in particular for tissue stimulating system
Dispositif de télémétrie, en particulier pour un système de stimulation des tissues

(30) Priorität: 19.05.1994 DE 4417927
(43) Veröffentlichungstag der Anmeldung: 07.02.1996
(73) Patentinhaber: BIOTRONIK Mess- und Therapiegeräte GmbH & Co Ingenieurbüro Berlin, 12359 Berlin (DE)
(72) Erfinder: Hastine, Allen K., Aurora, Oregon 97002 (US)
(74) Vertreter: Eisenführ, Speiser & Partner

(56) Entgegenhaltungen:
- US-A- 4 160 971
- US-A- 4 361 153
- US-A- 4 528 987
- US-A- 4 532 932
- US-A- 4 571 589

## Beschreibung

Die Erfindung betrifft eine passive Telemetrievorrichtung der im Oberbegriff des Anspruchs 1 angegebenen Art.

Eine solche Telemetrievorrichtung ist aus der Druckschrift US 4,223,679 A1 bekannt. Ihre funktionsbestimmenden Komponenten sind eine externe Sende-/Empfangseinrichtung mit einem Sender- und Empfängerschwingkreis und ein mit einem implantierten Gerät - etwa einem Gewebestimulator - verbundener LC- oder LR-Kreis ("impedance reflecting circuit"). Der interne und der externe Kreis sind über ihre einander benachbart angeordneten Induktivitäten magnetisch gekoppelt. Ein von dem implantierten Gerät nach außen zu übertragendes Signal wird über einen VCO in ein Spannungssignal umgewandelt, mit dem ein FET angesteuert wird. Dieser verändert die Impedanz des internen LC- oder LR-Kreises und verstimmt damit den Ausgang des mit diesem gekoppelten externen Oszillators.

Diese Vorrichtung weist den Nachteil auf, dass die Übertragungseigenschaften stark von der Anordnung der Spulen des internen Sende- und des externen Empfangskreises relativ zueinander abhängen und durch elektromagnetische Störfelder und metallische Fremdkörper sowie auch das Gehäuse des Stimulators stark beeinflußt werden. Die Übertragung funktioniert nur auf sehr kurze Distanzen von wenigen Zentimetern.

Aus EP 0 362 611 A1 ist eine Telemetrievorrichtung bekannt, die zwei externe Elektroden, die gemeinsam einen Sende- und Empfangsdipol bilden, aufweist und als Empfangselektroden die ventrikuläre und die indifferente Elektrode eines Herzschrittmachers nutzt. Hier erfolgt die Übertragung mittels elektrolytisch-galvanischer Kopplung über die intra- und extrazellulären Körperflüssigkeiten. Diese Vorrichtung erfordert zwei nicht zu nahe beieinander liegende interne Elektroden und ist ebenfalls relativ störanfällig.

Aus EP 0 179 536 A2 ist eine Anordnung zur Energie- und Signalübertragung zu implantierten Prothesen - im erläuterten Beispiel speziell einer Gehörprothese - bekannt, die insgesamt drei abgestimmte Spulen aufweist, von denen eine eine externe Sende-, die zweite eine interne Empfangsspule und die dritte mit der Sendespule gekoppelt ist und die Übertragungsverluste verringert. Auch bei dieser Anordnung müssen die extern und die intern angeordneten Spulen einander nahe benachbart sein und die Übertragung ist anfällig für äußere elektromagnetische Störungen. In der Druckschrift wird keinerlei Bezug zur passiven Telemetrie hergestellt.

Eine passive Telemetrievorrichtung , wie im Oberbegriff des Anspruchs 1 definiert mit einem impedanzmodulierten Transponder im Implantat ist aus der US 4,361,153 bekannt.

Der Erfindung liegt die Aufgabe zugrunde, eine passive Telemetrievorrichtung der eingangs genannten Gattung so weiterzubilden, daß die Lage- und Störempfindlichkeit entscheidend verringert wird.

Diese Aufgabe wird durch eine Vorrichtung mit den Merkmalen des Anspruchs 1 gelöst.

Die Erfindung schließt den Gedanken ein, sowohl eine Sende- als auch eine Empfangsantenne eines passiven Telemetriesystems für ein implantierten Gerät in fester, vorteilhaft gewählter Lagebeziehung zueinander außerhalb des Körpers anzuordnen, wobei beim implantierten Gerät innerhalb des Körpers ein Element vorgesehen ist, über das die elektromagnetischen Eigenschaften (komplexer Widerstand bzw. Leitfähigkeit) des Übertragungskanals geändert werden können. Dieses Element wirkt aber nicht selbst als Sender oder Empfänger und auch nicht als ein einen externen Schwingkreis verstimmendes Element.

Durch dieses Prinzip lassen sich die sich aus Veränderungen der Relativposition von Sender und Empfänger bei bekannten Anordnungen ergebenden Störungen nahezu völlig ausschalten und der Einfluß äußerer elektromagnetischer Felder kann erheblich verringert werden.

Im einzelnen weist die Telemetrievorrichtung eine Phasenschieberschaltung, eine mit deren Ausgang und einem Steuersignalausgang der Sendeeinheit verbundene (somit über ein Signal von der Sendeeinheit getriggerte) Sample-and-Hold-Schaltung und einen mit deren Ausgang verbundenen Schwellwertdetektor auf. Mit dieser Anordnung werden konstante Signalanteile ausgelöscht, die etwa vom metallischen Gehäuse eines Schrittmachers herrühren, und aus dem Empfangssignal werden die über die implantierte Spule modulierten Anteile extrahiert.

Das Element zur Veränderung der elektromagnetischen Eigenschaften der Übertragungsstrecke ist insbesondere eine mit einem Signalausgang des implantierten Teiles verbundene Spule mit in Abhängigkeit von ausgegebenen Signalen veränderbarem komplexem Widerstand. Diese in der Nähe der externen Sende- und Empfangsspule implantierte Spule moduliert in Abhängigkeit von ihr zugeführten Signalen den Widerstand bzw. die Leitfähigkeit der Sender-Empfänger-Strecke.

Wenn in einer vorteilhaften Ausbildung der Sende- und der Empfangsspule eine induktive Abstimmvorrichtung zugeordnet ist, mittels derer eine Auslöschung des von der Sendespule im Raumbereich der Empfangsspule erzeugten Magnetfeldes vorgenommen werden kann, kann in einem bestimmten Anfangszustand etwa Stromlosigkeit in der Empfangsspule eingestellt werden. Erfolgt dann eine Modulation des (komplexen) Widerstandes der Übertragungsstrecke, wird wieder ein Strom induziert, dessen Größe ein Maß für die Widerstandsänderung der implantierten Spule ist und somit die dieser zugeführten Signale reflektiert.

Die Abstimmvorrichtung für die Sende-/Empfangseinrichtung weist etwa eine oder mehrere veränderbare Drahtschleife(n) bzw. -windung(en) auf, wobei etwa einige "feste" Wicklungen zum Grobabgleich und eine bewegliche Schleife zur Feinabstimmung vorgesehen sein können.

Die Sende- und Empfangsspule sind in vorteilhafter Ausbildung jeweils ringförmig, insbesondere in Gestalt eines Kreises, Ovals oder Rechtecks, ausgebildet und konzentrisch zueinander im wesentlichen in derselben Ebene angeordnet. Daneben ist aber eine Vielzahl weiterer geometrischer Ausbildungen und Anordnungen möglich, wenn nur eine magnetische Kopplung zwischen beiden gewährleistet ist. Die Spule können auch in aufeinander senkrecht stehenden Ebenen gewickelt sein, und es können mehrere, in verschiedenen Ebenen angeordnete, Sende- und/oder Empfangsspulen vorgesehen sein.

In einer vorteilhaften Ausbildung weist die Empfangseinheit einen Triggersignaleingang auf, der mit einem Triggersignalausgang der Sendeeinheit verbunden ist, wodurch anhand eines Vergleiches der Phase des Empfangssignals mit dem Triggersignal Phasenverschiebungen auf der Übertragungsstrecke nachgewiesen werden können. Damit lassen sich in einfacher Weise Modulationen durch die implantierte Spule nachweisen und Störungen durch metallische Fremdkörper oder elektromagnetische Störfelder weitgehend unterdrücken.

Zur Eliminierung des Einflusses von Amplitudenänderungen kann die Empfangs- und Auswertungseinheit Mittel zum selektiven Sampling umfassen, wodurch ein ausschließlicher Nachweis von Phasenverschiebungen im Empfangssignal erfolgen kann.

Zur Signalübermittlung nach außen weist der implantierte Teil insbesondere Mittel zur Umsetzung der zu übermittelnden Information in Änderungen des komplexen Widerstands der mit seinem Ausgang verbundenen Spule - speziell Mittel zur analogen Umsetzung (Phasenmodulation) und/oder eine Vorrichtung zur digitalen Verschlüsselung (Pulscodemodulation) auf. Auf der Empfängerseite sind entsprechende Demodulations- bzw. Entschlüsselungsmittel vorzusehen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: ein stark schematisiertes Blockschaltbild einer Ausführungsform der Erfindung,
- Figur 2: eine Darstellung der geometrischen Anordnung der Sende- und Empfangsspule bei einer Ausführungsform der Erfindung und
- Figur 3: ein vereinfachtes Blockschaltbild der Sende- und Empfangseinheit bei einer speziellen Ausführungsform der Erfindung.

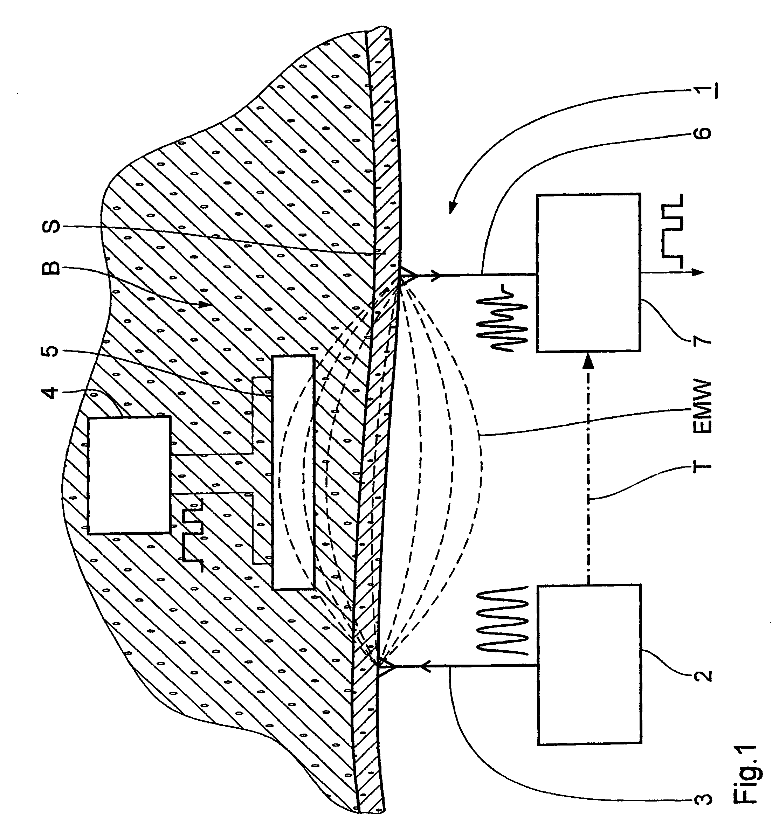

Fig. 1 zeigt schematisch eine Telemetrieanordnung 1, mit der das Prinzip der Erfindung verdeutlicht wird. Eine Sendeeinheit 2, in der sinusförmige elektromagnetische Schwingungen mit einer Frequenz von einigen bis zu einigen zehn kHz mit konstanter Amplitude erzeugt werden, ist mit einer auf oder nahe der Hautoberfläche S eines Körpers B angeordneten Sendeantenne 3 verbunden, über die diese Schwingungen als elektromagnetisches Wellenfeld EMW abgestrahlt werden.

Im Körperinneren B befindet sich ein implantiertes Teil - etwa ein Gewebestimulator - 4, das eine nahe der Hautoberfläche S und in der Nähe der Sendeantenne 3 angeordnetes steuerbares Element 5 aufweist, über das die elektromagnetischen Eigenschaften (insbesondere der Widerstand bzw. die Leitfähigkeit) des umgebenden Körpergewebes und damit die Ausbreitungsbedingungen für das elektromagnetische Wellenfeld EMW verändert werden können. Von dem implantierten Teil kann das steuerbare Element etwa - wie in der Figur symbolisch dargestellt - mit Impulsfolgen angesteuert werden, womit eine entsprechende Modulation des Wellenfeldes EMW bewirkt werden kann.

Wiederum auf oder nahe der Hautoberfläche ist eine Empfangsantenne 6 angeordnet, über die die elektromagnetischen Wellen EMW empfangen und das - in der Figur symbolisch als modulierte Sinusschwingung dargestellte - Empfangssignal einer Empfangseinheit 7 zugeleitet werden können. In der Einheit 7 erfolgt unter Zuhilfenahme eines vom Sender 2 gelieferten Steuer- oder Triggersignals T eine Verarbeitung, in deren Ergebnis - wie in der Figur durch den Rechteckimpulszug beim Element 7 verdeutlicht - ein dem von dem implantierten Teil 4 abgegebenen Signal adäquates Signal extern bereitsteht.

Die Anordnung kann dazu dienen, Informationen von einem implantierten Gewebestimulator - etwa ein intrakardiales EKG von einem Herzschrittmacher mit Mitteln zur Erfassung der Herzaktionspotentiale, Batteriezustandsinformationen, Typkennungen o.a. - nach außen zu übertragen, wo sie dem Arzt zur Analyse zur Verfügung stehen, gespeichert werden können etc. Ebensogut ist sie - unabhängig von einem Gewebestimulator - zur Übertragung von Meßwerten von implantierten Sensoren nach außerhalb des Körpers geeignet.

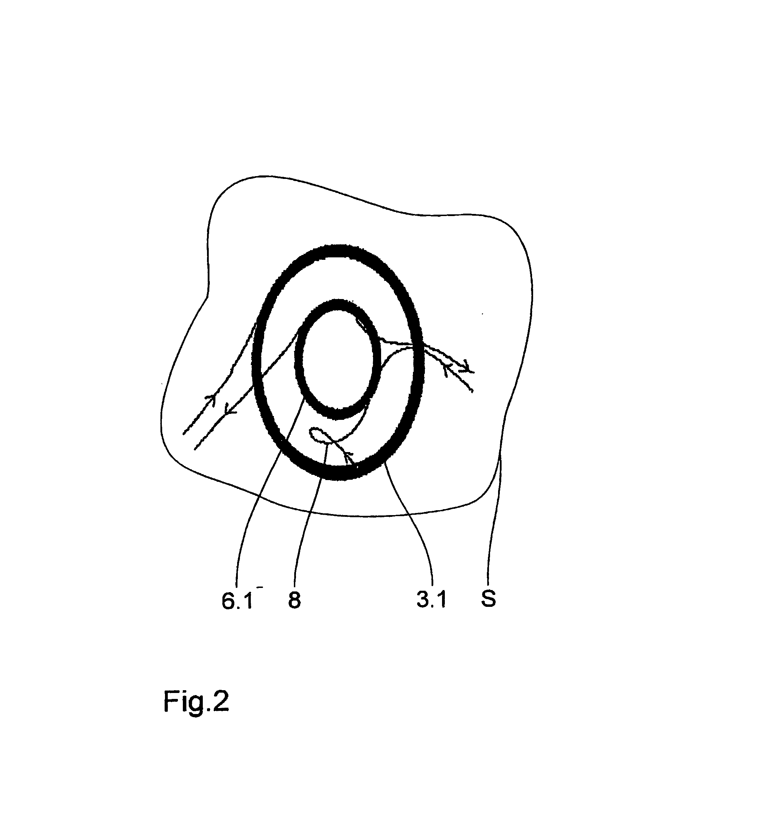

Fig. 2 zeigt ein Ausführungsbeispiel für eine auf einem Bereich der Hautoberfläche S eines Patienten aus Kupferdraht gewickelte Antennen-Anordnung 3.1, 6.1 in Gestalt zweier konzentrischer, elliptischer Spulen, von denen die innere - die Empfangsspule 6.1 - eine kleine Achse von etwa 2 cm Länge und die äußere - die Sendespule 3.1 - eine kleine Achse von etwa 4 cm Länge aufweist. Die Sendespule 3.1 hat im Beispiel eine Induktivität von 31 mH, die Empfangsspule eine solche von 35 mH.

Zur Auslöschung des von der Sendespule um Inneren der Empfangsspule erzeugten Magnetfeldes im abgeglichenen Zustand der Anordnung sind einige Windungen Draht der Sendespule mit entgegengesetzter Wicklungsrichtung um die Empfangsspule gewickelt. Mittels einer kleinen Schleife 8 erfolgt ein Feinabgleich der Anordnung, bis der Pegel des Empfangssignals bei Abwesenheit metallischer Gegenstände mindestens 40 dB unterhalb des Pegels des Sendesignals liegt.

Die Lage und Form der als Sende- bzw. Empfangsantenne dienenden Spulen kann gegenüber Fig. 2 in verschiedenartigster Weise abgewandelt sein: Eine kreisrunde oder ovale Form ist ebenso möglich wie eine drei- oder mehreckige (insbesondere auch rechteckige) oder etwa eine D-ähnliche Form etc. und die Spulen müssen nicht in derselben Ebene liegen. Die Auswertung übertragender Signale wird jedoch wesentlich erleichtert, wenn ein Abgleich derart möglich ist, daß bei Abwesenheit metallischer Gegenstände und in einem stationären Anfangszustand des implantierten Teils keine nennenswerte Kopplung zwischen beiden Antennen (Spulen) besteht, d. h. die Empfangsantenne bzw. -spule im wesentlichen stromlos ist.

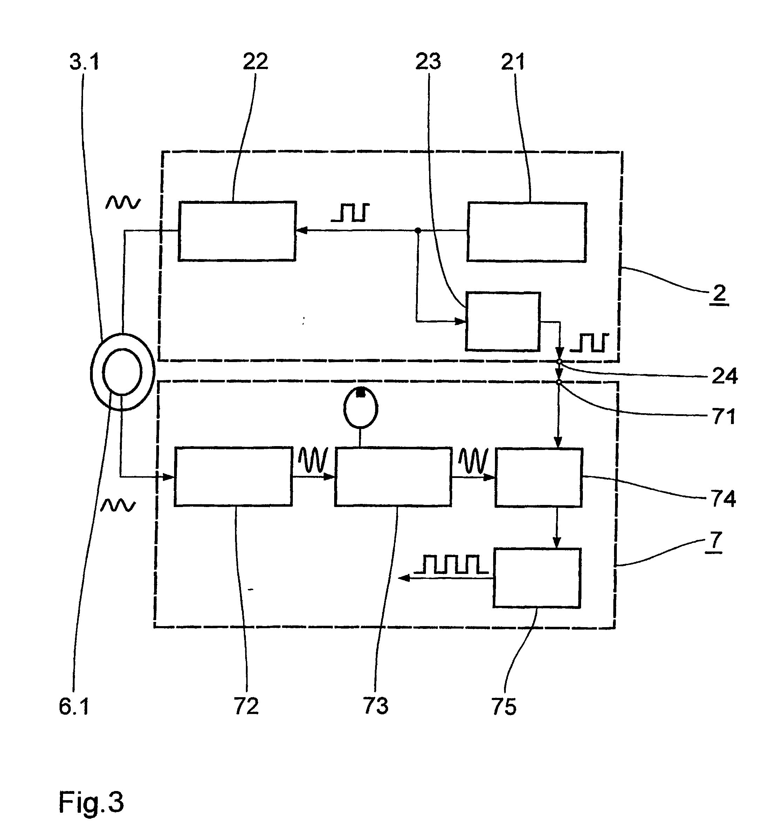

Fig. 3 zeigt ein vereinfachtes Blockschaltbild einer Sendeeinheit 2 und einer Empfangseinheit 7 mit der in Fig. 2 näher gezeigten Spulenanordnung 3.1, 6.1 in einer Ausführungsform der Erfindung, wobei Stromversorgungs-, Filter-, Bedien- u.a. Elemente weggelassen sind.

Die Sendeeinheit 2 weist einen Taktgenenator 21 auf, der Rechteckimpulse erzeugt. Dessen Ausgang ist mit den Eingängen eines Sinuswellengenerators 22 und eines Einzelimpulserzeugers ("one-shot") 23 verbunden. Von ersterem werden die erzeugten Schwingungen mit einer Frequenz von 15,625 kHz der Sendespule 3.1 zugeführt, über die sie als elektromagnetische Wellen abgestrahlt werden. Von letzterem werden Einzelimpulse als Triggersignal T einem Steuersignalausgang 24 und von dort einem Steuereingang 71 der Empfangseinheit 7 zugeführt.

Die Empfangseinheit 7 weist einen Eingangsverstärker 72 mit hoher Verstärkung (etwa 100 oder mehr) auf, dessen Ausgang mit einer Phasenschieberschaltung 73 verbunden ist. An dieser erfolgt eine Justierung der Nullpunktslage des verstärkten Empfangssignals derart, daß der Einfluß des Metallgehäuses eines (nicht gezeigten) implantierten Schrittmachers auf das der Weiterverarbeitung zugeführte Signal eliminiert ist, d.h. nur der modulierte Anteil des Empfangssignals weiterverarbeitet wird.

Dieses gelangt vom Ausgang der Phasenschieberschaltung 73 zum Signaleingang einer Sample-and-Hold-Schaltung 74, die auch mit dem Steuersignaleingang 71 verbunden ist und über diesen das Triggersignal T empfängt. Diese erfaßt zu den Triggerzeitpunkten jeweils die Amplitude des phasenverschobenen Signals, die die im implantierten Teil aufgeprägte Modulation repräsentiert. Das Ausgangssignal der Sample- and-Hold-Schaltung 74 wird einem Schwellwertdetektor 75 zugeführt, der es in digitale Signale umwandelt und diese zur weiteren Auswertung ausgibt.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

Insbesondere sind grundsätzlich auch andere, aus der Nachrichtentechnik als solche bekannte Antennen und Empfangsschaltungen für Frequenzen im kHz-Bereich und analog und/oder digital modulierte Signale einsetzbar.

## Patentansprüche

1. Telemetrievorrichtung, insbesondere für ein Gewebestimulator-System oder einen Körperzustandssensor, mit einem in einen Körper (B) implantierten Teil (4, 5) sowie einem außerhalb des Körpers angeordneten Teil (2, 3, 6, 7), einem Sende- und einem Empfangselement (3, 6; 3.1, 6.1), einer mit dem Sendeelement (3; 3.1) verbundenen Sendeeinheit (2) zur Erzeugung elektromagnetischer Schwingungen oder Impulse und einer mit dem Empfangselement (6; 6.1) verbundenen Empfangseinheit (7) zur Verarbeitung empfangener elektromagnetischer Schwingungen oder Impulse, wobei die Sendeeinheit (2) mit dem als Sendeantenne (3; 3.1) ausgebildeten Sendeelement und die Empfangseinheit (7) mit dem als Empfangsantenne (6; 6.1) ausgebildeten Empfangselement außerhalb des Körpers angeordnet sind, wobei Sende- und Empfangsantenne in einer festen Lage zueinander angeordnet sind, und der in den Körper implantierte Teil ein Element (5) zur Veränderung der elektromagnetischen Eigenschaften der Übertragungsstrecke zwischen Sende- und Empfangsantenne aufweist, **dadurch gekennzeichnet, daß** die Empfangseinheit (7) eine Phasenschieberschaltung (73), eine mit deren Ausgang und einem Steuersignalausgang (24) der Sendeeinheit (2) verbundene Sample-and-Hold-Schaltung (74) und einen mit deren Ausgang verbundenen Schwellwertdetektor (75) aufweist.

2. Telemetrievorrichtung nach Anspruch 1, **dadurch gekennzeichnet, daß** die Sende- und die Empfangsantenne als Spulen (3.1, 6.1) ausgebildet sind.

3. Telemetrievorrichtung nach Anspruch 1 oder 2, **dadurch gekennzeichnet, daß** das Element (5) zur Veränderung der elektromagnetischen Eigenschaften der Übertragungsstrecke eine mit einem Signalausgang des implantierten Teiles (4) verbundene Spule mit in Abhängigkeit von ausgegebenen Signalen veränderbarem komplexem Widerstand ist.

4. Telemetrievorrichtung nach einem der vorangehenden Ansprüche, **dadurch gekennzeichnet, daß** der Sende- und der Empfangsspule (3.1, 6.1) eine induktive Abstimmvorrichtung (8) zugeordnet ist, mittels derer eine Auslöschung des von der Sendespule im Raumbereich der Empfangsspule erzeugten Magnetfeldes vorgenommen werden kann.

5. Telemetrievorrichtung nach Anspruch 4, **dadurch gekennzeichnet, daß** die Abstimmvorrichtung eine oder mehrere bewegbare Drahtschleife(n) bzw.-windung(en) (8) aufweist.

6. Telemetrievorrichtung nach einem der vorangehenden Ansprüche, **dadurch gekennzeichnet, daß** die Sende- und Empfangsspule (3.1, 6.1) jeweils ringförmig, insbesondere in Gestalt eines Kreises, Ovals oder Rechtecks, ausgebildet und konzentrisch zueinander im wesentlichen in derselben Ebene angeordnet sind.

7. Telemetrievorrichtung nach einem der vorangehenden Ansprüche, **dadurch gekennzeichnet, daß** mehrere, in verschiedenen Ebenen angeordnete, Sende- und/oder Empfangsspulen vorgesehen sind.

8. Telemetrievorrichtung nach einem der vorangehenden Ansprüche, **dadurch gekennzeichnet, daß** die Empfangseinheit (7) einen Triggersignaleingang (7.1) aufweist, der mit einem Triggersignalausgang (24) der Sendeeinheit (2) verbunden ist, wodurch anhand eines Vergleiches der Phase des Empfangssignals mit dem Triggersignal Phasenverschiebungen auf der Übertragungsstrecke nachgewiesen werden können.

9. Telemetrievorrichtung nach einem der vorangehenden Ansprüche, **dadurch gekennzeichnet, daß** die Empfangseinheit (7) Mittel zum selektiven Sampling zum Nachweis von Phasenverschiebungen im Empfangssignal unter Eliminierung des Einflusses von Amplitudenänderungen aufweist.

10. Telemetrievorrichtung nach einem der vorangehenden Ansprüche, **dadurch gekennzeichnet, daß** der implantierte Teil (4) Mittel zur Umsetzung von aus dem implantierten Teil nach extern zu übermittelnder Information in Änderungen des komplexen Widerstands der mit seinem Ausgang verbundenen Spule (5) aufweist.

11. Telemetrievorrichtung nach Anspruch 10, **dadurch gekennzeichnet, daß** die Mittel zur Umsetzung Mittel zur analogen Umsetzung und/oder eine Vorrichtung zur digitalen Verschlüsselung umfassen.

## Claims

1. A telemetry device, in particular for a tissue stimulator system or a body condition sensor, comprising a portion (4, 5) which is implanted in a body (B) and a portion (2, 3, 6, 7) arranged outside the body, a transmitting and a receiving element (3, 6; 3.1, 6.1), a transmitting unit (2) connected to the transmitting element (3; 3.1) for producing electromagnetic oscillations or pulses and a receiving unit (7) connected to the receiving element (6; 6.1) for processing received electromagnetic oscillations or pulses, wherein the transmitting unit (2) with the transmitting element in the form of a transmitting antenna (3; 3.1) and the receiving unit (7) with the receiving element in the form of a receiving antenna (6; 6.1) are arranged outside the body, wherein the transmitting and receiving antennas are arranged in a fixed position relative to each other, and the portion implanted in the body has an element (5) for altering the electromagnetic properties of the transmission path between the transmitting and receiving antennas, **characterised in that** the receiving unit (7) has a phase shift circuit (73), a sample-and-hold circuit (74) connected to the output of the phase shift circuit and to a control signal output (24) of the transmitting unit (2), and a threshold value detector (75) connected to the output of the sample-and-hold circuit.

2. A telemetry device according to claim 1 **characterised in that** the transmitting and the receiving antennas are in the form of coils (3.1, 6.1).

3. A telemetry device according to claim 1 or claim 2 **characterised in that** the element (5) for altering the electromagnetic properties of the transmission path is a coil connected to a signal output of the implanted portion (4), with a complex resistance which is variable in dependence on outputted signals.

4. A telemetry device according to one of the preceding claims **characterised in that** associated with the transmitting and the receiving coils (3.1, 6.1) is an inductive tuning device (8), by means of which quenching of the magnetic field produced by the transmitting coil in the spatial region of the receiving coil can be effected.

5. A telemetry device according to claim 4 **characterised in that** the tuning device has one or more movable wire loops or turns (8).

6. A telemetry device according to one of the preceding claims **characterised in that** the transmitting and receiving coils (3.1, 6.1) are each of a ring-shaped configuration, in particular in the form of a circle, oval or rectangle, and are arranged concentrically relative to each other substantially in the same plane.

7. A telemetry device according to one of the preceding claims **characterised in that** there are provided a plurality of transmitting and/or receiving coils arranged in different planes.

8. A telemetry device according to one of the preceding claims **characterised in that** the receiving unit (7) has a trigger signal input (7.1) which is connected to a trigger signal output (24) of the transmitting unit (2) whereby phase shifts on the transmission path can be detected by means of a comparison of the phase of the reception signal with the trigger signal.

9. A telemetry device according to one of the preceding claims **characterised in that** the receiving unit (7) has means for selective sampling for the detection of phase shifts in the reception signal with elimination of the influence of changes in amplitude.

10. A telemetry device according to one of the preceding claims **characterised in that** the implanted portion (4) has means for converting information to be communicated to the exterior from the implanted portion into changes in the complex resistance of the coil (5) connected to its output.

11. A telemetry device according to claim 10 **characterised in that** the converting means include means for analog conversion and/or a device for digital encoding.

## Revendications

1. Dispositif de télémétrie, notamment pour un système de stimulation des tissus ou un capteur de l'état d'un corps, comportant une partie (4, 5) implantée dans un corps (B), ainsi qu'une partie (2, 3, 6, 7) disposée à l'extérieur du corps, un élément d'émission et un élément de réception (3, 6; 3.1, 6.1) d'une unité d'émission (2) reliée à l'élément d'émission (3; 3.1) et servant à produire des oscillations électromagnétiques ou des impulsions, et une unité de réception (7) reliée à l'élément de réception (6; 6.1) pour traiter des oscillations électromagnétiques ou des impulsions reçues, dans lequel l'unité d'émission (2) et l'unité de réception (7) sont disposées, respectivement avec l'élément d'émission agencé sous la forme d'une antenne d'émission (3; 3.1) et avec l'élément de réception agencé sous la forme d'une antenne de réception (6; 6.1) à l'extérieur du corps, l'antenne d'émission et l'antenne de réception sont disposées dans des positions fixes l'une par rapport à l'autre et la partie implantée dans le corps comporte un élément (5) servant à modifier les caractéristiques électromagnétiques de la section de transmission entre l'antenne d'émission et l'antenne de réception, **caractérisé en ce que** l'unité de réception (7) comporte un circuit de déphasage (73), un circuit d'échantillonnage et de maintien (74) relié à la sortie de ce circuit et une sortie (24) du signal de commande de l'unité d'émission (2), et un détecteur à valeur de seuil (75) relié à la sortie du circuit d'échantillonnage et de maintien.

2. Dispositif de télémétrie selon la revendication 1, **caractérisé en ce que** l'antenne d'émission et l'antenne de réception sont agencées sous la forme de bobines (3.1, 6.1).

3. Dispositif de télémétrie selon la revendication 1 ou 2, **caractérisé en ce que** l'élément (5) servant à modifier les caractéristiques électromagnétiques de la section de transmission est une bobine qui est reliée à une sortie des signaux de la partie implantée (4) et possède une résistance complexe pouvant être modifiée en fonction de signaux délivrés.

4. Dispositif de télémétrie selon l'une des revendications précédentes, **caractérisé en ce qu'**à la bobine d'émission et à la bobine de réception (3.1, 6.1) est associé un dispositif inductif de réglage d'accord (8), à l'aide duquel est réalisée une suppression du champ magnétique produit par la bobine d'émission dans la zone d'espace de la bobine de réception.

5. Dispositif de télémétrie selon la revendication 4, **caractérisé en ce que** le dispositif de réglage d'accord comporte une ou plusieurs boucles de fils ou spires de fils mobiles (8).

6. Dispositif de télémétrie selon l'une des revendications précédentes, **caractérisé en ce que** la bobine d'émission et la bobine de réception (3.1, 6.1) sont réalisées chacune avec une forme annulaire, notamment sous la forme d'un cercle, d'un ovale ou d'un rectangle, et sont disposées concentriquement l'une par rapport à l'autre essentiellement dans le même plan.

7. Dispositif de télémétrie selon l'une des revendications précédentes, **caractérisé en ce qu'**il est prévu plusieurs bobines d'émission et/ou de réception disposées dans des plans différents.

8. Dispositif de télémétrie selon l'une des revendications précédentes, **caractérisé en ce que** l'unité de réception (7) comporte une entrée de signaux de déclenchement(7.1), qui est reliée à une sortie (24) du signal de déclenchement de l'unité d'émission (2), ce qui a pour effet que des déphasages dans la section de transmission peuvent être décelés sur la base d'une comparaison de la phase du signal de réception au signal de déclenchement.

9. Dispositif de télémétrie selon l'une des revendications précédentes, **caractérisé en ce que** l'unité de réception (7) comporte des moyens d'échantillonnage sélectif pour déceler des déphasages dans le signal de réception moyennant l'élimination de l'influence de variations d'amplitude.

10. Dispositif de télémétrie selon l'une des revendications précédentes, **caractérisé en ce que** la partie implantée (4) comprend des moyens pour convertir une information, qui doit être transmise de la partie implantée en direction de l'extérieur, en des variations de la résistance complexe de la bobine (5) reliée à sa sortie.

11. Dispositif de télémétrie selon la revendication 10, **caractérisé en ce que** les moyens de conversion comportent des moyens de conversion analogique et/ou un dispositif de codage numérique.
